# EUROPEAN PATENT APPLICATION

(11) **EP 2 592 089 A1**
(43) Date of publication of application: **15.05.2013**
(21) Application number: 11188759.2
(22) Date of filing: 11.11.2011
(51) Int. Cl.: C07K 9/00, C07K 1/36, A61K 38/14

(54) **Process of purification of teicoplanin**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The invention relates to a process for purification of teicoplanin, in particular to a process for an efficient removal of impurities by means of adsorption of particular dissolved components from the filtrate of fermentation broth of *Actinoplanes teichomyceticus* culture on commercial hydrophobic or slightly polar adsorbent resins with further selective elution of the product from the resin. According to the invention, the process involves the use of a mixture of a water-miscible organic solvent in an aqueous solution at various steps of the purification process, such as in the above mentioned elution step and/or in an ultrafiltration step on membranes with a cut-off of 3-100kDa. The process further involves treating a teicoplanin solution with charcoal, e.g. after elution and before ultrafiltration. Preferably, the water-miscible organic solvent is selected from the group consisting of C₁-C₄ alcohols, C₃-C₄ ketones or C₂-C₄ nitriles.

## Description

### Field of the Invention

The present invention relates to the field of chemical technology, more specifically to a novel and effective way of purification of the antibiotic teicoplanin.

### Background of the invention

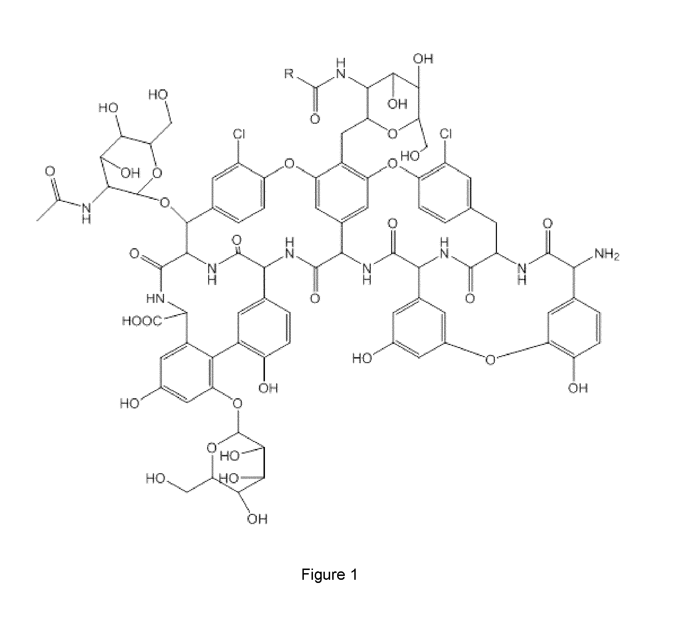

Teicoplanin is a commonly used term for a group of glycopeptide antibiotics from the vancomycin-ristocetin family, produced by a fungal microorganism such as e.g. *Actinoplanes teichomyceticus.* Teicoplanin is firstly disclosed in J. Antibiotics 31 (1978), p. 170-177 as a mixture of three factors named teichomycin A₁, A₂ and A₃. Factor A₂, later called teicoplanin A₂, was identified as the major component, which was isolated by column chromatography on Sephadex LH-20™. Teicoplanin A₂ was later recognized as a mixture of five molecules (J. Antibiotics 37 (1984), p. 615-620), which were soon elucidated as structures differing in the nature of an acyl-aliphatic side chain (J. Am. Chem. Soc. 106 (1984), 4895-4902). An exemplified and typical compound representation of teicoplanin is illustrated in Fig. 1 (R may denote various C₁₀-C₁₁-alkyl residues). These five A₂ compounds constitute teicoplanin together with another major component (A₃) and some minor components (e.g. Rₛ-1 to Rₛ-4).

The mixture of these components in a limited ratio variation is marketed as Targocid® for treating infections by gram-positive bacteria. Besides vancomycin and vancomycin-like antibiotics it still remains a key active compound against highly resistent microorganisms.

A number of efforts have been made to separate teicoplanin from a culture broth of the microorganism and to purify teicoplanin to reach pharmaceutical grade. According to the first publication in US 4239751, a culture broth is divided into a mycelial cake and a filtrate. After treatment of the cake both fractions were extracted with butanol at acidic pH and the crude fermentation product was precipitated after partial removal of the solvent. The precipitates were collected, purified on Sephadex LH20 column and on an acidic ion exchange resin, such as Amberlite IR-120 and Dowex 50, with final isolation of product by precipitation at 4°C. However, this process is a laboratory trial of isolation with several disadvantages for industrial use in view of complexity, quality, costs and yields.

In order to improve yields of fermentation in such self-inhibited production of teicoplanin the product was tried to be continuously removed by adding water-miscible organic solvents, such as acetone, n-propanol, and acetonitrile (Korean Pat. No. 367890), or by adding a strongly acidic cation exchange resin, such as Dow XFS-43278.00 and Diaion SK-1 02 (Korean Pat. No. 118034) to a culture broth. But such processes still need large volumes of organic solvents to extract teicoplanin causing handling problems and environmental pollution.

US patent application 4,845,194 reports a process for purification of teicoplanin involving a cation exchange technique without prior pH adjustment. Teicoplanin is eluted from the cation exchange resin using a solution having a pH value of from about 9 to about 12.

Further progress in the isolation of teicoplanin was made by discovery of the efficient binding of teicoplanin to hydrophobic resins, firstly disclosed in EP 0241758, using a special polyamide resin and further upgrading the purification by an extraction process of teicoplanin from mycelium at alkaline pH as a simplification of primary disclosed processes (EP 0479086). However, the purity of teicoplanin was not higher than 85 % and the decolourisation was insufficient.

The introduction of commercial hydrophobic resins, preferably applied as a column rather than in suspension treatment, represented a further progress. The publication J. Biochem. 275 (2000), p. 6201-6206 describes the use of a commercial hydrophobic resin Dianion HP2 MGL™ with further elution using NaOH solution. However, the approach required the continuous neutralization of the eluting solution in order to prevent epimerisation of the alkali-sensitive product. Even if accompanied with other special approaches in order to obtain pure teicoplanin, in view of a mere 50 to 60 % (w/w) of purity this laboratory process is rather suitable for purposes other than use as a pharmaceutical ingredient.

Korean Pat. No. 321304 discloses a neutral adsorption resin and a lectin-immobilized affinity chromatography step using resins selected from the group consisting of Amberlite XAD 16, Diaion HP 20, silica gel, and activated carbon. However, in case that the filtered culture broth extracted from a basic solution is adsorbed to a resin, such as Diaion HP 20, a substantial amount of teicoplanin is lost in the adsorption step, and the purity of teicoplanin eluted by a methanol concentration gradient is very low. Moreover, it is necessary to remove methanol in order to apply the solution to a lectin-immobilized resin. Furthermore, it is not desirable to apply the strategy in large scale production because of the cost of lectin-immobilized resins. According to Korean pat. appl. 2003/017067 teicoplanin of a culture broth is adsorbed to a porous adsorption resin, the porous adsorption resin is washed with diluted hydrochloric acid and teicoplanin is desorbed from the adsorption resin using a mixed solution of water and acetone. The eluting solution containing teicoplanin is concentrated by vacuum distillation, treated with an activated carbon, and subjected to a precipitation process, and thereby teicoplanin is purified. Again, the process is insufficient for a scale up due to the irreversible adsorption of teicoplanin to the resin reducing the yield and due to the use of large amounts of solvent . Furthermore, it is difficult to purify teicoplanin to 95 area % or higher employing only a single process that uses porous adsorption resins.

Alternatively, some other studies have included reverse phase resins to separate and purify teicoplanin from the culture broth. For example, a process, published in Chromatographia 24 (1987) p. 295-301 proposed purifying teicoplanin using a Lichrosorb™ resin, but the use of large amounts of acetonitrile and the frequent exchange of resin may considerably enhance production costs. Two other alternatives accomplished the chromatographic methods on reverse phase resins by additional manners of purification. For example, Korean pat. appl. 2003/092504 describes extraction of fermentation products with aqueous acetone followed by chromatography on silane coated silica gel, adsorption on activated carbon in the step of isolating and ultra-filtration. US 7405267 recites a process of purifying teicoplanin from a culture broth, which includes a primary purifying step using a synthetic adsorbent, a secondary purifying using a cation-exchange resin, a catalytic resin, or a chelate resin, a tertiary purifying step using a reverse phase resin, and a final lyophilisation step. Accordingly, the use of more expensive reverse phase alone cannot avoid the use of additional adsorbents, so it does not justify the additional costs. In summary, all above described methods offer only partial solutions, which do not solve the complexity of isolation and purification of teicoplanin. The patent EP 1735337B1 has offered the most comprehensive solution for isolation and purification of teicoplanin until now. It went back to the adsorption method on hydrophobic resins describing the following key steps of purification and isolation of teicoplanin:
- separation of mycelium from dissolved teicoplanin in a filtrate,
- adsorption of roughly purified teicoplanin to porous adsorption resins from neutral 10-40 % aqueous C₁-C₄-alcohols or C₃-C₄-ketones medium of pH 6.5 to 7.5, followed by elution with 40 to 90 % aqueous C₁-C₄-alcohols with careful salt concentration control,
- adsorption of impurities to activated carbon using diluted aqueous alcohol solution, roughly calculated from aqueous methanol with max. 30 %
- desalination by adsorption to porous resins followed by elution with 40 to 90 % aqueous C₁-C₄-alcohols
- ultrafiltration on membranes with pores size from 3 - 100 kDa at 0 - 4 kPa of pressure using diluted aqueous alcoholic solutions of purified teicoplanin in concentration less than 20 %
- precipitation of teicoplanin.

Although this process may represent the best literature option of isolation of teicoplanin, its reproduction surprisingly shows considerable drawbacks:
- desorption from porous resins are not complete (maximally 87.6 %);
- diminishing of size pores in ultrafiltration surprisingly reduces passage of teicoplanin through membranes although the pores are considerably larger than teicoplanin molecules radius, while using larger pores reduces selectivity, so several cycles of ultrafiltration is needed enhancing solvent medium amount to huge volumes;
- removal of coloured impurities remains the main problem, because colours are not completely removed neither in the step adsorption on resins nor during decolourisation on charcoal and ultrafiltration.

Hence, there is still a need for an efficient comprehensive procedure of isolation of teicoplanin of pharmaceutical grade.

### Summary of the invention

The present invention provides the following aspects, subject-matters and preferred embodiments, which respectively taken alone or in combination, further contribute to solving the object of the present invention:
1. A process for preparing purified teicoplanin, the process comprising the steps of
   a) adsorbing teicoplanin to a hydrophobic adsorption resin;
   b) desorbing teicoplanin from said resin using a medium containing water and a water-miscible organic solvent;
   c) treating a solution comprising teicoplanin with charcoal followed by charcoal removal;
   d) subjecting a solution comprising teicoplanin to a process of ultrafiltration on membranes with a cut-off of 3-100 kDa, wherein the solution contains more than 20 % (v/v) of a water-miscible organic solvent and not more than 90 % (v/v) of a water-miscible organic solvent;
   e) optionally concentrating and/or desalinating a teicoplanin-containing solution after ultrafiltration defined in step d) by ultrafiltration on membranes with a cut-off of 1000 Da;
   f) obtaining purified teicoplanin.
2. The process according to item 1, wherein the steps a) to f) are performed in the order as indicated in item 1.
3. The process according to items 1 or 2, further comprising in advance a step of adding an organic solvent to a culture of *Actinoplanes teichomyceticus,* and removal of mycelium and other insoluble material by filtration and/or centrifugation.
4. The process according to any one of items 1 or 3, wherein the hydrophobic adsorption resin is a polystyrene or polyacrylate crosslinked non-ionic macroreticular resin having a pore size of 20 to 500 Å.
5. The process according to any one of items 1 to 4, wherein the hydrophobic adsorption resin has a pore size of 100 to 500 Å.
6. The process according to any one of items 1 to 5, wherein the hydrophobic adsorption resin is selected from the group consisting of Hypersol-Macronet®, Dowex® Optipore, Amberlite® XAD16, Amberlite® XAD 1600, Amberlite® XAD 1180, Diaion® HP20, Diaion® Sepabeads SP207 and Dianion® HP2MG.
7. The process according to any one of items 1 to 6, wherein the hydrophobic adsorption resin is selected from the group consisting of Amberlite® XAD16, Amberlite® XAD 1600 and Amberlite® XAD 1180.
8. The process according to any one of items 1 to 7, wherein the adsorption to the hydrophobic adsorption resin is performed in a solution comprising 30 % (v/v) or less of a water-miscible solvent.
9. The process according to any one of items 1 to 8, wherein the step of desorbing teicoplanin from the hydrophobic adsorption resin is performed using an aqueous medium comprising at least 40 % (v/v) of a water-miscible organic solvent.
10. The process according to any one of items 1 to 9, wherein the water-miscible organic solvent is selected from C₁-C₄ alcohols, C₃-C₄ ketones or C₂-C₄ nitriles.
11. The process according to any one of items 1 to 10, wherein the water-miscible solvent is methanol.
12. The process according to any one of items 1 to 11, wherein the step of desorbing teicoplanin from the hydrophobic adsorption resin is performed using an aqueous medium comprising 60-70 % (v/v) of methanol.
13. The process according to any one of items 1 to 12, wherein the step of desorbing teicoplanin from the hydrophobic adsorption resin is performed using an aqueous medium having a pH value below 4.
14. The process according to item 13, wherein the aqueous medium has a pH value of 2 to 2.5.
15. The process according to items 12 or 14, wherein the pH value of the aqueous medium is adjusted by addition of an aqueous solution of a mineral acid, an organic acid or an acidic buffer.
16. The process according to any one of items 13 to 15, wherein the pH value of the aqueous medium is adjusted by addition of hydrochloric acid, sulphuric acid, phosphoric acid or citric acid.
17. The process according to any one of items 1 to 16, wherein the solution obtained in step b) is concentrated before charcoal treatment of step c).
18. The process according to any one of items 1 to 17, wherein the pH value of the solution obtained in step b) is adjusted to pH of 6.0 - 8.0 by adding a base selected from inorganic hydroxides, carbonates, phosphates or by adding a buffer before charcoal treatment.
19. The process according to any one of items 1 to 18, wherein the medium of the solution undergoing the charcoal treatment originates from step b) and comprises at least 40 % (v/v) of an organic solvent, selected from the group of C₁-C₄ alcohols, C₃-C₄ ketones or C₂-C₄ nitriles.
20. The process according to any one of items 1 to 19, wherein the solution obtained in step b) comprises 60 to 80 % (v/v) of methanol or is corrected by addition of fresh solvent in order to have a methanol concentration of 60 to 80 % (v/v).
21. The process according to any one of items 1 to 20, wherein the weight ratio between teicoplanin and active charcoal in the solution is from 1 : 0.05 to 1 : 2, preferably from 1 : 0.2 to 1 : 1, most preferably from 1 :0.5 to 1: 0.8.
22. The process according to any one of items 1 to 21, wherein the ultrafiltration of step d) is performed on a solution obtained in step c), which comprises more than 20 % (v/v) and up to 90 % (v/v) of a C₁-C₄ alcohol.
23. The process according to any one of items 1 to 22, wherein the ultrafiltration of step d) is performed on a solution obtained in step c), which has been corrected by addition of fresh solvent in order to comprise more than 20 % (v/v) and up to 90 % (v/v) of a C₁-C₄ alcohol.
24. The process according to any one of items 1 to 23, wherein the ultrafiltration of step d) is performed on a solution obtained in step c) comprising 40-80 % (v/v) of methanol, preferably 60-80 % (v/v) of methanol.
25. The process according to any one of items 1 to 24, wherein the ultrafiltration of step d) is performed using a membrane with a cut-off of 3 - 100 kDa, preferably of 5 - 100 kDa, more preferably of 5 - 10 kDa.
26. The process according to any one of items 1 to 25, wherein the ultrafiltration of step d) is characterized by a retentate/permeate ratio of teicoplanin concentrations, which does not exceed 0.65, more preferably does not exceed 0.4.
27. The process according to any one of items 1 to 26, wherein the ultrafiltration of step e) is performed using membranes with a cut-off of 1000 Da.
28. The process according to any one of items 1 to 27, wherein obtaining purified teicoplanin in step f) is performed by teicoplanin isolation involving removal of the solvent using evaporation, vacuum evaporation, liophilisation, spray drying or precipitation by adding antisolvent.
29. The process according to item 28, wherein acetone is used as an antisolvent.
30. The process according to item 28 or 29, wherein the precipitated teicoplanin is isolated by filtration or centrifugation and optionally dried in vacuo.
31. The process according to any one of items 1 to 30, wherein the purified teicoplanin is for pharmaceutical use.
32. A process for manufacturing a pharmaceutical composition comprising the process according to any one of items 1 to 31, and wherein purified teicoplanin is admixed with at least one pharmaceutical excipient or carrier.

### Brief description of the drawings

- Figure 1:: Illustrates an exemplified and typical compound representation of teicoplanin.
- Figure 2:: Schematically shows a typical deamination of teicoplanin.
- Figure 3:: Provides a schematic overview of an exemplary process according to the invention.
- Figure 4:: Shows the portion of adsorbed teicoplanin as a ratio of the concentration at a given time point to the initial concentration.
- Figure 5:: Desorption of teicoplanin and related impurities from Amberlite XAD16™ adsorbent resin with neutral water-methanol mixtures: the dependence of the concentration of desorbed teicoplanin (circles) and the part of coloured related impurities, expressed as an area % compared to teicoplanin (squares), from the concentration of methanol is shown in the diagram as a result of two sets of experiments.
- Figure 6:: Desorption of teicoplanin and its impurities from Amberlite XAD1600™ adsorbent resin with neutral water-methanol mixtures: the dependence of the concentration of desorbed teicoplanin (circles) and the part of coloured related impurities, expressed as an area % compared to teicoplanin (squares), from the concentration of methanol is shown in the diagram as a result of two sets of experiments
- Figure 7:: Desorption of teicoplanin and its impurities from Amberlite XAD1180™ adsorbent resin with neutral water-methanol mixtures: The dependence of the concentration of desorbed teicoplanin (circles) and the part of coloured related impurities, expressed as an area % compared to teicoplanin (squares), from the concentration of methanol is shown in the diagram.
- Figure 8:: Desorption of teicoplanin and its impurities from Amberlite XAD16™ adsorbent resin with acidic water-methanol mixtures: The dependence of the concentration of desorbed teicoplanin (circles) and the part of coloured related impurities, expressed as an area % compared to teicoplanin (squares), from the concentration of methanol at different pH of the eluant (pH = 2 - full line, pH = 3 - hatched line, pH = 4 - dotted line) is shown in the diagram.
- Figure 9:: Desorption of teicoplanin and its impurities from Amberlite XAD1600™ adsorbent resin with acidic water-methanol mixtures: the dependence of the concentration of desorbed teicoplanin (circles) and the part of coloured related impurities, expressed as an area % compared to teicoplanin (squares) from the concentration of methanol at different pH of the eluant (pH = 2 - full line, pH = 3 - hatched line, pH = 4 - dotted line) is shown in the diagram.
- Figure 10:: Desorption of teicoplanin and its impurities from Amberlite XAD1180™ adsorbent resin with acidic water-methanol mixtures: the dependence of the concentration of desorbed teicoplanin (circles) and the part of coloured related impurities, expressed as an area % compared to teicoplanin (squares), from the concentration of methanol at different pH of the eluant (pH = 2 - full line, pH = 3 - hatched line, pH = 4 - dotted line) is shown in the diagram.
- Figure 11:: Teicoplanin solution decolourization with activated carbon in water-methanol mixtures: yield (circles) and relative absorbance reduction (squares) in dependence from methanol concentration are shown in the diagram.
- Figure 12:: Teicoplanin solution decolourization with activated carbon in various loads: yield (circles) and relative absorbance reduction (squares), in dependance from various loads of charcoal, expressed as a w/w relation to teicoplanin, are shown in the diagram.
- Figure 13:: Teicoplanin permeability through ultrafiltration membranes in different methanol/water mixtures: samples of the permeate and of the concentrate were taken and analyzed by HPLC.

### Detailed description of the invention

The present invention is now described in more detail by preferred embodiments and examples, which are however presented for illustrative purpose only and shall not be understood as limiting the scope of the present invention in any way.

Therefore the present invention is to provide an inexpensive process for isolation of teicoplanin from a fermentation culture of a fungal microorganism producing teicoplanin such as e.g. *Actinoplanes teichomyceticus* in a grade for pharmaceutical application with substantial removal of colours.

The term "teicoplanin" as used in the context of the invention relates to a glycopeptide antibiotic that consists of a mixture of compounds or to the single components of said mixture. The single components of the mixture typically comprise five major components (A₂-1, A₂-2, A₂-3, A₂-4, A₂-5), a core glycopeptide A₃-1 and minor components such as RS-1, RS-2, RS-3 and RS-4. Explicit reference is made to the description of the background of the invention as cited herein. The content ratio of the single components may not be constant and can vary according to the used strain, culturing conditions or the purification process applied.

The fermentation of teicoplanin produces an intensely brown to almost black coloured broth, which can be gradually decolourised through a selected technological procedure by adsorption and chromatographic methods known to a skilled person, in order to obtain a final product that is still considerably coloured in yellow or brownish cast, which can additionally darken by storage. We surprisingly found that the coloured impurities belong to at least three physicochemical groups, which cannot be removed by only one technical operation. Critical impurities can be categorized into the following groups.

One group represents high molecular weight coloured impurities, which can be removed by methods of separation suitable for large molecules, such as adsorption to resins or gel filtration. They mainly comprise macromolecular residuals of cell structure.

Another group represents low molecular impurities, probably less than 1000 Da, of lower polarity, that also originated unspecific cell components comprising molecules with double bonded lipid parts.

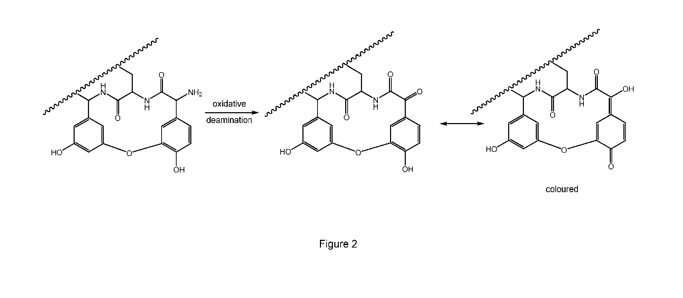

The third group represents closely related degradation products, characterised in the publication J. Antibiotics 49 (1996), p. 644-650, supposedly produced by oxidative aminolysis. Replacement of the amino group by a keto group causes a double effect: the affected part of the molecule becomes a conjugative quinone like structure, moving the absorption maximum to the visual spectrum and the molecule loses basic character (Figure 2).

Thus, the invention provides a process for an efficient removal of impurities by means of adsorption of particular dissolved components from the filtrate of fermentation broth of a culture of a teicoplanin producing fungal microorganism, preferably a *Actinoplanes teichomyceticus* culture, on commercial hydrophobic or slightly polar adsorbent resins with further selective elution of the product from the resin. According to the invention, the process involves the use of a mixture of a water-miscible organic solvent in an aqueous solution at various steps of the purification process. Preferably, the water-miscible organic solvent is selected from the group consisting of C₁-C₄ alcohols, C₃-C₄ ketones or C₂-C₄ nitriles.

The filtrate of fermentation broth of *Actinoplanes teichomyceticus* culture herein means a filtered culture broth without mycelium prepared by procedures known to a skilled biotechnologist.

In a preferred embodiment, the process comprises the steps of
a) adsorbing teicoplanin to a hydrophobic adsorption resin;
b) desorbing teicoplanin from said resin using a medium containing water and a water-miscible organic solvent;
c) treating a solution comprising teicoplanin with charcoal followed by charcoal removal;
d) subjecting a solution comprising teicoplanin to a process of ultrafiltration on membranes with a cut-off of 3-100 kDa, wherein the solution contains more than 20 % (v/v) of a water-miscible organic solvent and not more than 90 % (v/v) of a water-miscible organic solvent;
e) optionally concentrating and/or desalinating a teicoplanin-containing solution after ultrafiltration defined in step d) by ultrafiltration on membranes with a cut-off of 1000 Da;
f) finally obtaining purified teicoplanin.

Commercially available hydrophobic or slightly polar adsorbent resins can be selected from polystyrene or polyacrylate crosslinked non-ionic macroreticular resins of pore radius of 20 to 500 A, selected from, but not limited to, trademarks Hypersol-Macronet®, Amberlite® XAD, Diaion® HP and Sepabeads SP, or Dowex® Optipore, preferably selected from the resins of pore radius of 100 to 500 A selected from trademarks Amberlite® XAD16, Amberlite® XAD 1600, Amberlite® XAD 1180, Diaion® HP20, Diaion® Sepabeads SP207, Dianion® HP2MG, most preferably from Amberlite® XAD16, Amberlite® XAD 1600, Amberlite® XAD 1180. Resins of such type selectively adsorb molecules with lipophilic residues, including molecules of slightly larger molecular weight such as teicoplanin, from aqueous solutions and leave low molecular polar components including ionic solutes and a variety of macromolecules in the solution. Less polar molecules are adsorbed from aqueous solution, which contains no more than 30 % (v/v) of water miscible solvents, preferably no more than 20 % of C₁-C₄ alcohols, C₃-C₄ ketones or C₂-C₄ nitriles, preferably methanol.

The ease of adsorption depends on the type of resin; a less favourable adsorption may be compensated by longer time of treatment. Representative Example 2 shows kinetics of adsorption of teicoplanin to three typical resins: Amberlite® XAD16, Amberlite® XAD 1600, Amberlite® XAD 1180. All three resins show good adsorption, but some of them adsorb faster than others. Using Amberlite® XAD 1180, more than 90 % of the teicoplanin initially present is adsorbed from the solution in 2 hours, while with XAD16 only 50 % is adsorbed. A resin with larger pores (e.g. Amberlite® XAD 1180) adsorbs faster than one with smaller pore (e.g. Amberlite® XAD16), but this can be compensated by smaller particle and larger surface area (e.g. Amberlite® XAD 1600). Favourable kinetics leads to a more efficient column adsorption, which is important for efficient industrial scale production of teicoplanin.

From said solutions, the adsorbent removes at least 80 % of total teicoplanin, preferably at least 90 %, most preferably at least 95 % of total teicoplanin. The adsorption process is performed by shaking the resin suspended in said teicoplanin solution for at least 2 hours, preferably for at least 12 hours, most preferably for at least 24 hours at a temperature from 0-40 °C, preferably at room temperature. Finally, the teicoplanin containing resin is filtered off and optionally washed by water by resuspending or rinsing. Alternatively the teicoplanin containing filtered broth is eluted through a column of said adsorbent loading fermentation products onto the adsorbent up to the saturation.

In a preferred embodiment, the adsorbed material is released from a resin by eluting with aqueous solutions rich in organic solvent, preferably containing at least 40 % (v/v) of a water-miscible organic solvent, selected from C₁-C₄ alcohols, C₃-C₄ ketones or C₂-C₄ nitriles, preferably methanol. The extent of teicoplanin elution from a resin can be enhanced by addition of an organic solvent. However, the elution is not complete and not highly selective. Together with teicoplanin, also most of the impurities are eluted. The concentration of the organic solvent for elution of teicoplanin is limited by the solubility of teicoplanin in the organic solvent. For example, when applying methanol/water mixtures, the extent of tecoplanin elution reaches a maximum at concentrations of 60 - 80 % (v/v) of methanol depending on the type of resin, but the extent of elution is still lower than 60 % of total adsorbed tecoplanin. By enhancing ionic strength through addition of salts, 87.6 % of teicoplanin can be eluted (EP 1735337B1), but the selectivity remains insufficient or even decreases; especially removal of coloured impurities is still poor. Generally, adding a base to the elution system (J. Biochem. 275 (2000), p. 6201-6206) is another solution, but the approach cannot be followed in the specific case of teicoplanin since teicoplanin is not stable in alkaline media.

According to the invention, the elution medium is preferably acidified, preferably by mineral acid, most preferably by hydrochloric acid. It was surprisingly found that the efficiency of teicoplanin elution is higher than when using neutral elution methods described in the prior art. Even more, the approach according to the present invention enhances selectivity in view of removal of coloured impurities derived from said oxidative deamination. Examples 3-5 supplemented by corresponding figures show elution efficiencies of teicoplanin and its oxidative impurities from three typical resins (Amberlite® XAD 16, XAD1600 and XAD 1180) as a function of organic solvent concentration in neutral medium of pH values 6 - 7, while Examples 6-8 show similar trials in an acidic medium at pH values 2, 3 and 4, respectively. It is evident that in neutral medium the relative concentration of coloured impurities reaches 7-12 % of concentration of teicoplanin as measured in area % of HPLC analysis at the organic solvent concentration, where desorption of teicoplanin has its maximum. The relative concentration of said impurities in acidic medium does not exceed 6 %, at pH 2 does not exceed 4 %. Two to three times lower levels of impurities are considerably easier to remove quantitatively in the following steps of teicoplanin isolation. Furthermore, teicoplanin is far more stable in acidic conditions than in alkaline media.

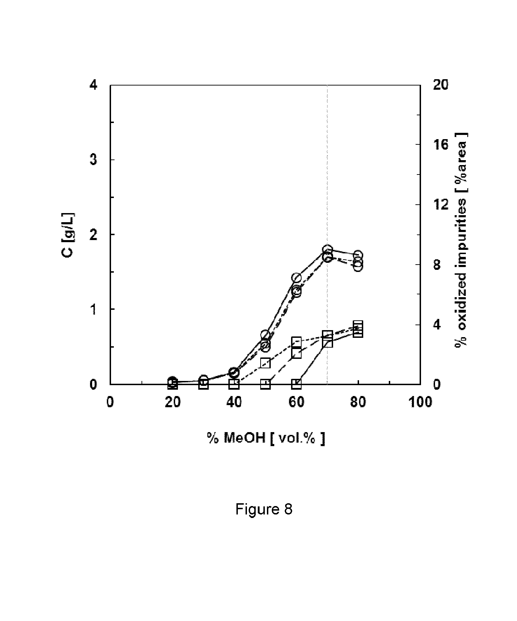

Examples 6-8 and Figures 8-10 illustrate beneficial effects of an acidic medium on the extent of teicoplanin release and the selectivity in view of coloured impurities in a batch process. When elution is performed with 60 % (v/v) organic solvent (methanol), the impurity content in eluted teicoplanin at a pH value of 2 is below 3 %, but when the pH value increases to 3 or 4 at the same elution conditions, impurity content increases up to 8 %. Teicoplanin of pharmaceutical grade is allowed to have a maximum of 5 % of impurities according to Ph. Eur. 7.2.

The separation of teicoplanin from coloured impurities can be improved by continuous elution from a column packed with a teicoplanin loaded resin, when the process is led similarly to a chromatographic process in which teicoplanin is eluted first, followed by elution of said impurities. Therein, the selectivity depends on the point of side fraction cut-off. A better selectivity usually reduces the yield. Nevertheless, the yield/selectivity ratio could be improved by using longer columns, thus reducing the relative content of impurities below 4 %, preferably below 2%, most preferably below 1 % in relative area % as determined via HPLC.

Thus, according to a preferred embodiment of the invention teicoplanin is eluted from a polystyrene or polyacrylate crosslinked non-ionic macroreticular resin having a pore radius of 20 to 500 Å, preferably selected from Amberlite® XAD16, Amberlite® XAD 1600, Amberlite® XAD 1180 by an aqueous medium containing at least 40 % (v/v) of a water-miscible organic solvent, selected from C₁-C₄ alcohols, C₃-C₄ ketones or C₂-C₄ nitriles, preferably methanol, most preferably containing 60-70% (v/v) of methanol, at a pH value below 4, preferably at a pH value of 2 - 2.5. The pH value is regulated by addition of an aqueous solution of mineral or organic acid, preferably selected from hydrochloric acid, sulphuric acid, phosphoric or citric acid or by acidic buffer.

In another preferred embodiment of the invention a solution containing teicoplanin molecules is treated by active charcoal, wherein the solution contains at least 40 % (v/v) of a water-miscible organic solvent. The starting solution is preferably previously purified by adsorption/desorption on hydrophobic adsorption resins, more preferably by desorption with acidic media. In a most preferred case the solvent/water ratio is not corrected before the charcoal treatment, i.e. the solution of teicoplanin is directly transferred from the previous step only by adjusting the pH value to neutral interval of 6-8.

The combined treatment with adsorption resin and charcoal has been described in EP 1735337B1. According to the description therein, a methanolic solution is preferably first diluted with water to achieve the solution with at most 40 % (v/v) of methanol by calculation and then treated with charcoal. This step is also obvious for the skilled person knowing that non-polar compounds are better adsorbed to charcoal from more polar media. The inventors surprisingly found that such dilution with water is not necessary. The treatment can be continued without hydration of the medium without losing the efficiency of removal of lipophilic impurities, even more, removal of coloured impurities is more efficient in methanol-rich aqueous mixtures. Such behaviour is irregular, but hypothetically explained by the amphiphilic property of teicoplanin molecules, which can assemble in aqueous media to macromolecular micelles with a lipohilic core. Said core may be able to trap some lipophilic impurities preventing them to have a contact with charcoal surface.

Thus, in a preferred embodiment the improved process of purification and decolourisation of teicoplanin solution on active charcoal includes the treatment of a teicoplanin solution rich in organic solvents, containing at least 40 % (v/v) of an organic solvent, which is particularly selected from the group of C₁-C₄ alcohols, C₃-C₄ ketones or C₂-C₄ nitriles, preferably methanol, more preferably in 60 - 80 % (v/v) methanol. In a further preferred embodiment, the teicoplanin/charcoal weight ratio is from 1 : 0.05 to 1 : 2, preferably from 1 : 0.2 to 1 : 1, more preferably 1 : 0.5 to 1: 0.8. Therein, incubation is preferably performed for 5 minutes to 12 hours, preferably to 0.5 - 2 hours at 0-60 °C, preferably at room temperature. The active charcoal is then filtered off and the solution of teicoplanin with reduced yellow colour is further treated to isolate the solid product.

The improved charcoal treatment mainly reduces the levels of lipohilic low molecular coloured compounds and other impurities of lower polarity.

In a preferred embodiment, the teicoplanin solution, previously substantially purified from low molecular organic impurities, is further treated by ultrafiltration on membranes having a cut-off of 1 kDa to 100 kDa.

Ultrafiltration is a method frequently used for isolation of fermentation products. The method is primarily used to eliminate macromolecular impurities. This is especially important for pharmaceutical ingredients for parenteral use because such macromolecules may have pyrogenic activity. Methods of ultrafiltration use porous membranes, which allow for the passage of smaller molecules, while holding back high molecular weight molecules. The size of pores in the membrane is decisive with respect to which molecules pass the membrane (permeate solution) and which molecules are cut off (retentate solution). Membranes of 1 kDa to 100 kDa cut-off are usually used for ultrafiltration, depending on the purpose. Membranes with small pores, which are permeated mainly by solvents and by salts are used for concentration and desalination of retentate, while membranes with larger pores are used for removal of larger molecules, a process also termed diafiltration. Molecules having a molecular weight, which is close to the pore size, are distributed between permeate and retentate. In the case of high retentate/permeate concentration ratios, several cycles of diafiltration using fresh solvent can be run in order to reach an acceptable yield of the product in the permeate.

Teicoplanin molecules with molecular weight of close to 1900 Da are expected to penetrate easily membranes with pores larger than 5 kDA, so membranes with a cut-off of 5 kDa or 100 kDa should be useful for good separation of larger molecules from teicoplanin. A large part of macromolecules can be removed by a method of adsorption on unpolar resins as described above. However, this method does not offer a complete removal of larger molecules, thus leaving a considerable amount of potential pyrogens, macromolecular colours and other larger molecules in the solution. The application EP 1735337B1 describes ultrafiltration through membranes with 30 kDa or 50 kDa using solutions of teicoplanin with a maximum of 20 % (v/v) of methanol or isopropanol. Such a procedure may be insufficient in view of pyrogens removal, because 3 kDa to 10 kDa weight cut-off membranes are required for the removal of pyrogens . Furthermore it was found that the retentate/permeate concentration ratio is very high, when using membranes with a cut-off of 30 kDa to 50 kDa, e.g. more than 80 % even at 50 - 100 kDa membranes using diluted alcohol solution up to 20 % (v/v). Several cycles of diafiltration must be applied taking days and huge amounts of fresh solvents. Using smaller pores in order to obtain an apyrogenic product the ultrafiltration becomes almost unproductive when applying prior art methods. This observation is surprising and cannot be explained by the calculated molecular weight of teicoplanin, which is several times smaller than the average cut-off of membranes.

According to the invention, the solution of teicoplanin is treated by ultrafiltration through membranes with a cut-off of 3 - 100 kDa preferably of 5 - 10 kDa, wherein the solution contains more than 20 % (v/v) of a water-miscible organic solvent and not more than 90 % (v/v) of a water-miscible organic solvent, which is particularly selected from the group of C₁-C₄ alcohols, C₃-C₄ ketones or C₂-C₄ nitriles, preferably methanol, more preferably 40-80 % (v/v) of methanol, most preferably 60-80 % (v/v) of methanol. In a preferred embodiment, a membrane type of 5 - 10 KDa molecular weight cut-off and a preferred concentration of methanol 60-80 % (v/v) in a teicoplanin solution is used. The retentate/permeate ratio of teicoplanin concentration does not exceed 0.65, preferably does not exceed 0.4, wherein the permeate is visually without yellow colour. Preferably, the process does not need more than 6 cycles of diafiltration on 5 kDa membranes and does not need more than 4 cycles on 10 kDa membranes respectively in order to obtain over 95 % yield of teicoplanin in the permeate, wherein yellow colour is substantially removed. This process mainly removes macromolecular coloured impurities, and other macromolecular impurities including potential pyrogens. In a preferred embodiment, the method is combined with other methods to remove colours of other chemical classes.

The surprising benefit of the enhanced organic solvent concentration in the teicoplanin solution in the phase of ultrafiltration additionally indicates that teicoplanin molecules are not dissolved monomolecularly in water-rich solutions but they seem to be assembled to multimolecular clusters, which cannot permeate the pores of ultrafiltration membranes which are theoretically sufficiently large for one molecule, but not sufficiently large for clusters.

The obtained solution of teicoplanin is optionally further treated by ultrafiltration through 1000 Da cut-off membranes in order to desalinate and concentrate the solution. The solution may further be treated with an antisolvent preferably selected from the group of esters, nitriles or ketones, preferably acetone to give a white precipitate of teicoplanin of at least 95 area %, preferably at least 98 area %.

In a preferred embodiment, the overall process for preparation of teicoplanin with increased purity, which includes the removal of coloured materials of different molecular types and other products of fermentation and degradation from a fermentation broth of teicoplanin producing culture, according to the invention comprises the following steps:
i) preparing a solution of fermentation broth;
ii) adsorbing teicoplanin to a hydrophobic adsorption resin;
iii) desorbing teicoplanin from said resin by acidic medium containing water and a water-miscible solvent;
iv) treating the obtained solution with charcoal wherein the solution contains at least 40 %
(v/v) of organic solvent followed by charcoal removal;
v) submitting the solution to a process of ultrafiltration on membranes with with a cut-off of 3-100 kDa, wherein the solution contains more than 20 % (v/v) of organic solvent;
vi) optionally concentrating and desalinating the solution by ultrafiltration on membranes with a cut-off-of 1000 Da;
vii) isolating solid teicoplanin.

According to this embodiment, step i), includes killing a fungal microorganism producing teicoplanin, preferably a culture of *Actinoplanes teichomyceticus,* decomposing cell structures by adding an organic solvent and removal of mycelium and other insoluble products of fermentation by filtration or centrifugation to give a clear but deeply coloured brown or almost black solution.

The hydrophobic adsorption resin of step ii) is selected from the polystyrene or polyacrylate crosslinked non-ionic macroreticular resins of pore radius of 20 to 500 Å, selected from the group of trademarks Hypersol-Macronet®, Amberlite® XAD, Diaion® HP and Sepabeads SP, or Dowex® Optipore, preferably selected from the resins of pore radius of 100 to 500 A selected from trademarks Amberlite® XAD16, Amberlite® XAD 1600, Amberlite® XAD 1180, Diaion® HP20, Diaion® Sepabeads SP207, Dianion® HP2MG, most preferably from Amberlite® XAD16, Amberlite® XAD 1600, Amberlite® XAD 1180. The adsorption can be performed by adding a resin to the solution with further stirring or shaking for 1 - 48 hours, preferably for at least 2 hours, more preferably for at least 12 hours, most preferably for at least 24 hours at temperature from 0-40 °C, preferably at room temperature. Subsequently, the teicoplanin containing resin is filtered off and optionally washed with water by resuspending or rinsing. Alternatively, fermentation products are adsorbed by loading the solution on a column, filled with said resin followed by optional washing.

The desorption of step iii) can be performed by elution of the adsorbed material with an aqueous medium containing at least 40 % (v/v) of a water-miscible organic solvent, selected from the group of C₁-C₄ alcohols, C₃-C₄ ketones or C₂-C₄ nitriles, preferably methanol, most preferably containing 60-70% (v/v) of methanol, at a pH value below 4, preferably at a pH value of 2-2.5. The pH value is regulated by addition of an aqueous solution of mineral or organic acid, preferably selected from hydrochloric acid, sulphuric acid, phosphoric or citric acid or by addition of an acidic buffer.

In step iv) of this embodiment, the solution is first optionally concentrated and adjusted to a pH of 6-8 by adding a base selected from inorganic hydroxides, carbonates, phosphates or by adding a buffer and then treated by an active charcoal. The medium of the solution used for the treatment of this step can originate from the step iii), containing at least 40 % (v/v) of organic solvent, selected from the group of C₁-C₄-alkanol, acetone or acetonitrile, preferably selected from methanol, preferably in 60 - 80 % (v/v) methanol or is corrected by addition of fresh solvent to achieve said content of solvent. The amount of active charcoal in the solution is measured relatively to the assay of teicoplanin. The teicoplanin/charcoal weight ratio is between 1 : 0.05 to 1 : 2, preferably from 1 : 0.2 to 1 : 1, most preferably from 1 :0.5 to 1: 0.8. The solution is treated with charcoal for 5 minutes to 12 hours, preferably to 0.5 - 2 hours at 0-60 °C, preferably at room temperature. The active charcoal is then filtered off to obtain the solution of teicoplanin with reduced yellow colour.

The ultrafiltration of step v) is preferably performed using the solution of the previous step. The mixture can optionally be corrected by addition of fresh solvent in order to contain 20-90 % (v/v) of of a water-miscible organic solvent selected from the group of C₁-C₄ alcohols, C₃-C₄ ketones or C₂-C₄ nitriles, preferably methanol, more preferably to contain 40-80 % (v/v) of methanol, most preferably 60-80 % (v/v) of methanol. The membranes of this step are selected from the membranes with a cut-off of 3 - 100 kDa, preferably of 5 - 100 kDa, more preferably of 5 - 10 kDa. In a preferred embodiment, the membrane has a cut-off of 5 - 10 KDa and the concentration of methanol is 60-80 % (v/v). In the teicoplanin solution the retentate/permeate ratio of teicoplanin concentration does not exceed 0.65 preferably does not exceed 0.4.

The ultrafiltration of optional step vi) is preferably performed on the solution of the previous step using membranes having a cut-off of 1000 Da. Teicoplanin remains in the retentate, while some small molecules like solvents and inorganic salts permeate. The solution should be concentrated to reach the solvent ratio and teicoplanin concentration suitable in order to provide for a high yield in the next step.

The isolation of teicoplanin in step vii) can be suitably performed by removal of solvent using evaporation, vacuum evaporation, liophilisation, spray drying or precipitation by adding an antisolvent. The precipitation with an antisolvent is preferred, using the solution of the previous step as a basic solution and an organic solvent, preferably acetone, as an antisolvent. The precipitated product is isolated by filtration or centrifugation and dried in vacuo.

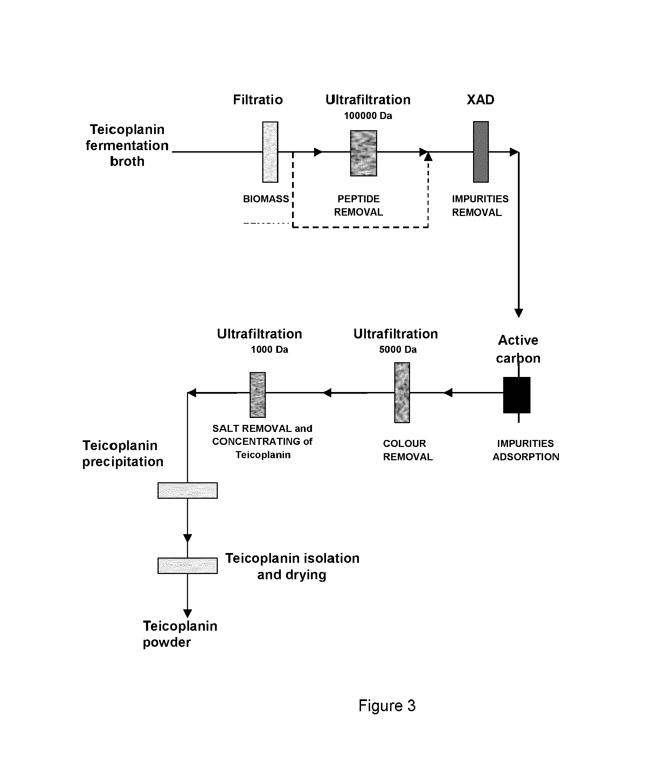

An overall process of an embodiment according to the present invention is schematically shown in Figure 3.

The preparation of a pharmaceutical composition comprising teicoplanin of high purity and pharmaceutically acceptable excipients is a further aspect of the present invention. The pharmaceutical composition of the invention is suitable for parenteral administration and the therapeutic dose of teicoplanin according to the invention in any given case will depend on the nature and severity of the disease to be treated. The dose and dose frequency may also vary according to the age, body weight, and response of the individual patient. In general, a suitable dose of the active ingredient is within the range of 50 mg to 800 mg daily, preferably 100 mg to 400 mg, preferably between 100 to 800 mg of total daily dosage. Dosage forms include dispersions, solutions, suspensions, emulsions, powders, implants, wherein the most preferred dosage forms of teicoplanin are dry powder injections and infusions. In a most preferred embodiment the novel teicoplanin described herein is dissolved in sterile and apyrogen water together with stabilisers, buffers, and additives, and the mixture is liophilised to give amorphous powder, which is reconstituted in prepared liquid just before use. The reconstituted injection may be administered either intravenously or intramuscularly. The intravenous injection may be administered, for example, as a bolus or as a 30 minute infusion. Dosage is usually once daily but, in cases of severe infection, a second injection should be administered on the first day in order to reach the required serum concentrations more rapidly.

### Examples

The following examples further illustrate the invention. They are provided for illustrative purposes only and are not intended to limit the invention in any manner.

The amount of teicoplanin components in various intermediate solutions or isolated solid intermediates and final products of representative experimental examples are measured by HPLC method on Agilent 1100™ instrument. The mobile phase A contains a 5 mM phosphate buffer of pH 4.9 and the mobile phase B contains acetonitrile. Chromatographic peaks were detected at 280 nm, temperature of column was 45 °C and 5 µl of sample were injected. The separation was performed on the column Ascentis Express C18™, 10 cm x 4.6 mm, 2.7 µm equipped with precolumn C18, 2 cm x 4.0 mm, 3 µm. Flow rate of 2.2 ml/min and the gradient during chromatography was as follows:

**Table1**

| Time (min) | Mobile phase B |
|---|---|
| 0.0 | 15% |
| 0.8 | 20 % |
| 0.9 | 24 % |
| 3.3 | 25 % |
| 4.8 | 29 % |
| 8.4 | 41 % |
| 8.7 | 60 % |
| 9.2 | 60 % |
| 9.5 | 15% |
| 11.0 | 15% |

Values are expressed as area percentage of the sum of all teicoplanin components or particular components themselves and have mainly relative or comparative meaning.

Transmitance is measured on Perkin Elmer Lamda 25 UV-VIS spectrometer at 405 nm and at concentration of teicoplanin in the samples of 40 g/l in water.

### EXAMPLE 1

### Isolation of crude fermentation broth

Fresh whole broth, collected immediately after the end of fermentation of *Actinoplanes teichomyceticus* was used for experiments. A broth supernatant (clarified broth) was produced by centrifuging the broth in a laboratory centrifuge (Megafuge 1.0R™, Heraeus) at 4000-6000 rpm for 10-30 minutes. The supernatant was additionally filtered through a sintered glass filter funnel (porosity 4 and 5, Schott Duran GmbH) and later analyzed by HPLC.

Clear filtrate (permeate) was produced by means of a cross-flow filtration. Ceramic filtration module (Membralox 1P19-60™) with 0.1 µm pore size was used. Permeate was collected and analyzed by HPLC.

### EXAMPLE 2

### Teicoplanin adsorption kinetics on Amberlite XAD16™, Amberlite XAD1600™ and Amberlite XAD1180™

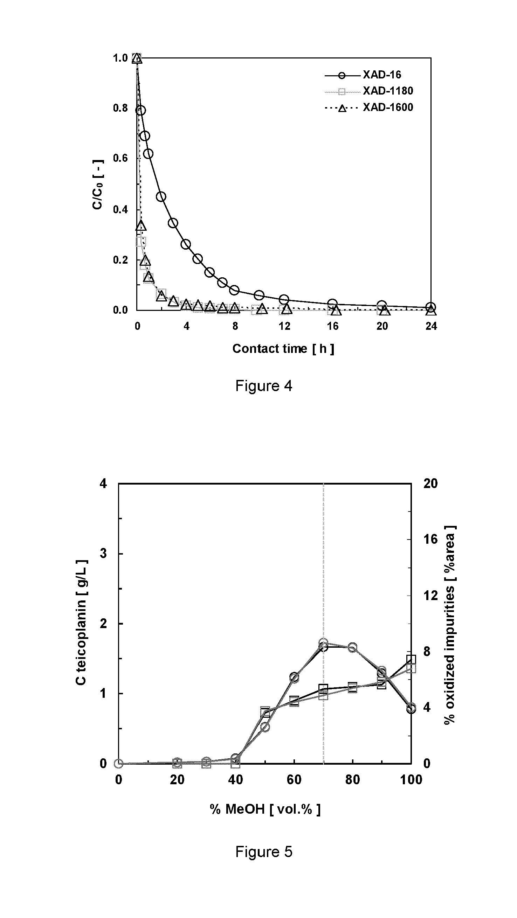

Equal amounts (4 g) of the adsorbents Amberlite XAD16™, Amberlite XAD1600™ and Amberlite XAD1180™ (trademark of Rohm & Haas) were weighed into 25 mL flasks, 15 parallels for each resin respectively. 20 mL of teicoplanin broth supernatant prepared as described in Example 1 were added to each flask. The flasks were vigorously shaken at room temperature on a laboratory shaker. Flasks were removed from the shaker in predefined time intervals (every hour in the first 8 hours, followed by every 2 hours in the next 4 hours and every 4 hours up to 24 hours). The slurry was filtered and samples of the filtrate were analyzed by HPLC. The portion of adsorbed teicoplanin is shown as a ratio of the concentration at a given time point to the initial concentration in the diagram (Figure 4).

### EXAMPLE 3

### Desorption of teicoplanin and related impurities from Amberlite XAD16™ adsorbent resin with neutral water-methanol mixtures

26,01 g of Amberlite XAD16™ resin (trademark of Rohm & Haas) were weighed into 1000 mL flask and 420 mL of teicoplanin broth filtrate were added. The flask was vigorously shaken on a laboratory shaker at room temperature. After 24 hours, the resin was filtered off and rinsed with 600 mL of demineralized water. The obtained teicoplanin containing resin was divided into ten 25 mL flasks ( 1 g to each one) followed by addition of a methanol/water mixture with 0, 20, 30, 40, 50, 60, 70, 80, 90, 100 % v/v ratio, each one into a particular flask. All flasks were vigorously shaken on the laboratory shaker at room temperature. After 24 hours, sample solutions were taken and analyzed by HPLC in view of teicoplanin and the corresponding coloured oxidised impurities. The dependence of the concentration of desorbed teicoplanin (circles) and the part of coloured related impurities, expressed as an area % compared to teicoplanin (squares), from the concentration of methanol is shown in the diagram as a result of two sets of experiments (Figure 5).

### EXAMPLE 4

### Desorption of teicoplanin and its impurities from Amberlite XAD1600™ adsorbent resin with neutral water-methanol mixtures

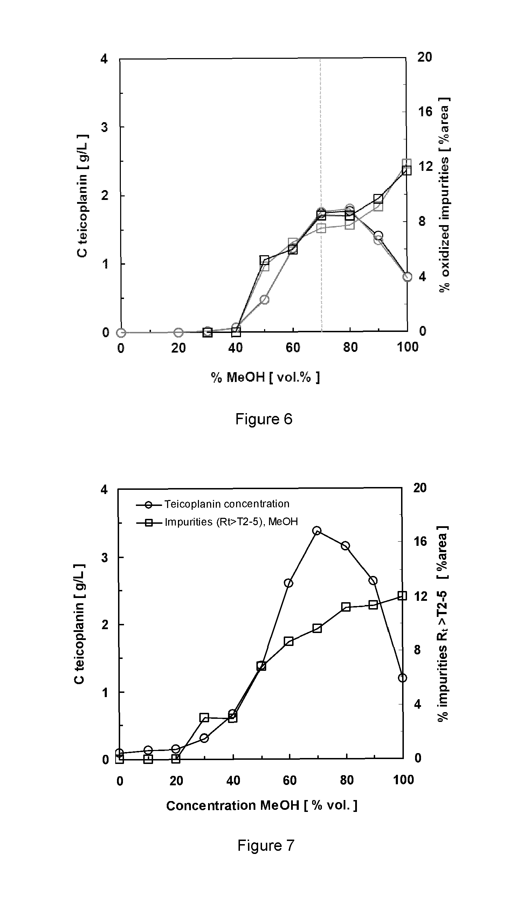

26,02 g of Amberlite XAD1600™ resin (trademark of Rohm & Haas) were weighed into a 1000 mL flask and 420 mL of teicoplanin broth filtrate were added. The flask was vigorously shaken on a laboratory shaker at room temperature. After 24 hours, the resin was filtered off and rinsed with 600 mL of demineralized water. The obtained teicoplanin containing resin was divided into ten 25 mL flasks ( 1 g to each one) followed by addition of a methanol/water mixture with 0, 20, 30, 40, 50, 60, 70, 80, 90, 100 % v/v ratio, each one into a particular flask. All flasks were vigorously shaken on the laboratory shaker at room temperature. After 24 hours, sample solutions were taken and analyzed by HPLC in view of teicoplanin and the corresponding coloured oxidised impurities. The dependence of the concentration of desorbed teicoplanin (circles) and the part of coloured related impurities, expressed as an area % compared to teicoplanin (squares), from the concentration of methanol is shown in the diagram as a result of two sets of experiments (Figure 6).

### EXAMPLE 5

### Desorption of teicoplanin and its impurities from Amberlite XAD1180™ adsorbent resin with neutral water-methanol mixtures

25 g of Amberlite XAD1180™ resin (trademark of Rohm & Haas) were weighed into a 1000 mL flask and 400 mL of teicoplanin broth filtrate were added. The flask was vigorously shaken on a laboratory shaker at room temperature. After 24 hours, the resin was filtered off and rinsed with 150 mL of demineralized water. The obtained teicoplanin containing resin was divided into eleven 25 mL flasks (1 g to each one) followed by addition of a methanol/water mixture with 0, 10, 20, 30, 40, 50, 60, 70, 80, 90, 100 % v/v ratio, each one into a particular flask. All flasks were vigorously shaken on the laboratory shaker at room temperature. After 24 hours, samples of the solutions were taken and analyzed by HPLC in view of teicoplanin and the corresponding coloured oxidised impurities. The dependence of the concentration of desorbed teicoplanin (circles) and the part of coloured related impurities, expressed as an area % compared to teicoplanin (squares), from the concentration of methanol is shown in the diagram (Figure 7).

### EXAMPLE 6

### Desorption of teicoplanin and its impurities from Amberlite XAD16™ adsorbent resin with acidic water-methanol mixtures

26 g of Amberlite XAD16™ resin (trademark of Rohm & Haas) were weighed into a 1000 mL flask and 440 mL of teicoplanin broth filtrate were added. The flask was vigorously shaken on a laboratory shaker at room temperature. After 24 hours, the resin was filtered off and rinsed with 600 mL of demineralized water. The obtained teicoplanin containing resin was divided into three sets of 25 mL flasks, each set with 7 flasks (1 g of the resin to each one), followed by addition of a methanol/water mixture with 20, 30, 40, 50, 60, 70, 80 % v/v ratio, each one into a particular flask of each set. The pH value of the particular set of flasks was adjusted to pH 2, 3 and 4 respectively. All flasks were vigorously shaken on a laboratory shaker at room temperature. After 24 hours, sample solutions were taken and analyzed by HPLC in view of teicoplanin and the corresponding coloured oxidised impurities. The dependence of the concentration of desorbed teicoplanin (circles) and the part of coloured related impurities, expressed as an area % compared to teicoplanin (squares), from the concentration of methanol at different pH of the eluant (pH = 2 - full line, pH = 3 - hatched line, pH = 4 - dotted line) is shown in the diagram (Figure 8).

### EXAMPLE 7

### Desorption of teicoplanin and its impurities from Amberlite XAD1600™ adsorbent resin with acidic water-methanol mixtures

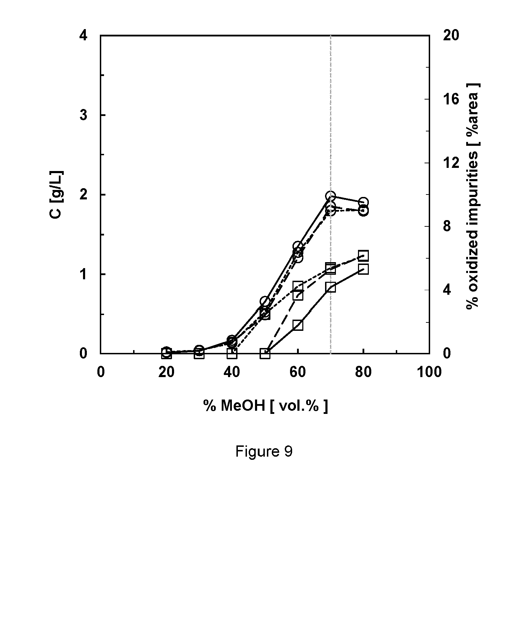

26.4 g of Amberlite XAD1600™ resin (trademark of Rohm & Haas) were weighed into a 1000 mL flask and 440 mL of teicoplanin broth filtrate were added. The flask was vigorously shaken on a laboratory shaker at room temperature. After 24 hours, the resin was filtered off and rinsed with 500 mL of demineralized water. The obtained teicoplanin containing resin was divided into three sets of 25 mL flasks, each set with 7 flasks (1 g of the resin to each one), followed by addition of a methanol/water mixture with 20, 30, 40, 50, 60, 70, 80 % v/v ratio, each one into a particular flask of each set. The pH value of the particular set of flasks was adjusted to pH 2, 3 and 4 respectively. All flasks were vigorously shaken on a laboratory shaker at room temperature. After 24 hours, sample solutions were taken and analyzed by HPLC in view of teicoplanin and the corresponding coloured oxidised impurities. The dependence of the concentration of desorbed teicoplanin (circles) and the part of coloured related impurities, expressed as an area % compared to teicoplanin (squares) from the concentration of methanol at different pH of the eluant (pH = 2 - full line, pH = 3 - hatched line, pH = 4 - dotted line) is shown in the diagram (Figure 9).

### EXAMPLE 8

### Desorption of teicoplanin and its impurities from Amberlite XAD1180™ adsorbent resin with acidic water-methanol mixtures

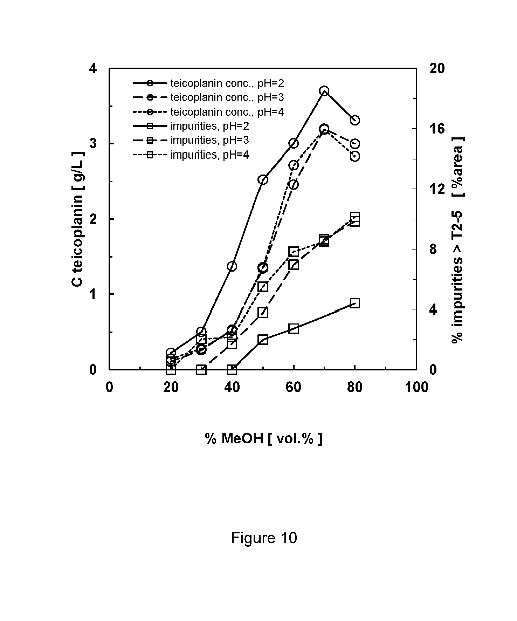

26.4 g of Amberlite XAD1180™ resin (trademark of Rohm & Haas) were weighed into a 1000 mL flask and 440 mL of teicoplanin broth filtrate were added. The flask was vigorously shaken on a laboratory shaker at room temperature. After 24 hours, the resin was filtered off and rinsed with 500 mL of demineralized water. The obtained teicoplanin containing resin was divided into three sets of 25 mL flasks, each set with 7 flasks (1 g of the resin to each one), followed by addition of a methanol/water mixture with 20, 30, 40, 50, 60, 70, 80 % v/v ratio, each one into a particular flask of each set. The pH value of the particular set of flasks was adjusted to pH 2, 3 and 4 respectively. All flasks were vigorously shaken on a laboratory shaker at room temperature. After 24 hours, sample solutions were taken and analyzed by HPLC in view of teicoplanin and the corresponding coloured oxidised impurities. The dependence of the concentration of desorbed teicoplanin (circles) and the part of coloured related impurities, expressed as an area % compared to teicoplanin (squares), from the concentration of methanol at different pH of the eluant (pH = 2 - full line, pH = 3 - hatched line, pH = 4 - dotted line) is shown in the diagram (Figure 10).

### EXAMPLE 9

### Teicoplanin solution decolorization with activated carbon in water-methanol mixtures

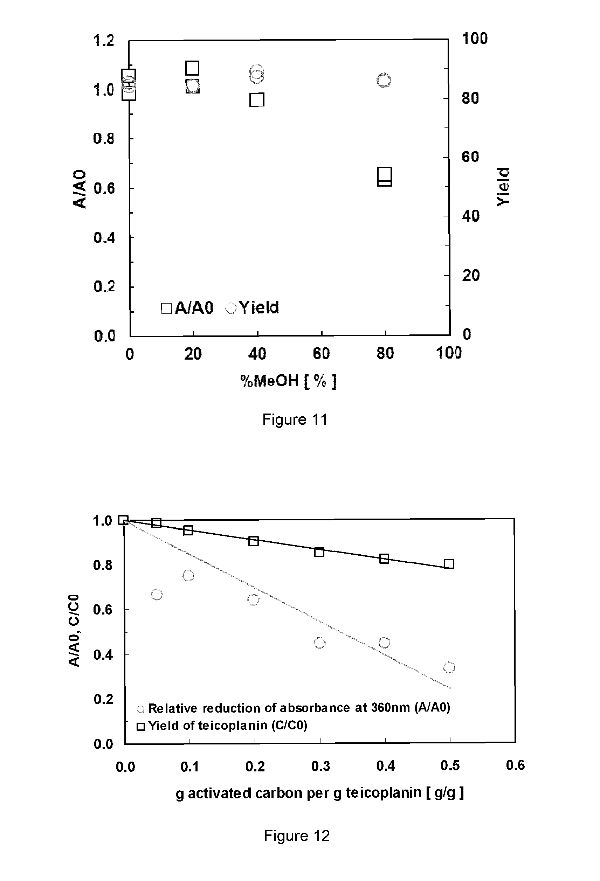

Activated carbon Norit CA1 was weighed (approx. 0.57 g activated carbon per g of teicoplanin) into ten 50 mL flasks. 20 mL of teicoplanin solution (4.5 g teicoplanin per litre in 0, 20, 40, 60 and 80 % vol. methanol-water mixture) were added into each flask. The flasks were vigorously shaken on a laboratory shaker at room temperature. After one hour, the slurry was filtered and samples were taken and analyzed by HPLC. The UV spectra of the samples were recorded by spectrophotometer. Yield (circles) and relative absorbance reduction (squares) in dependence from methanol concentration are shown in the diagram (Figure 11).

### EXAMPLE 10

### Teicoplanin solution decolorization with activated carbon in various loads

Various loads of activated carbon Norit CA1™ were weighed into six 25 mL flasks. 15 mL of teicoplanin solution (5 g teicoplanin per litre in 80 % vol. methanol-water mixture) were added into each flask. The flasks were vigorously shaken on a laboratory shaker at room temperature. After one hour, the slurry was filtered and samples were taken and analyzed by HPLC. The UV spectra of the samples were recorded by spectrophotometer. Yield (circles) and relative absorbance reduction (squares), in dependance from various loads of charcoal, expressed as a w/w relation to teicoplanin, are shown in the diagram (Figure 12).

### EXAMPLE 11

### Preparation of crude teicoplanin by column adsorption on adsorption resin

A lab scale column of 4.9 cm of diameter was filled with Amberlite XAD-1180™. The adsorbent bed height was 84 cm. The dynamic loading capacity of XAD-1180 was estimated to at least 65-70 g of teicoplanin per litre of adsorbent at 0.5 to 1 BV/h. 0.76 BV/h. 57.5 L of teicoplanin broth permeate, prepared as described in Example 1 and containing 1.9 g/L of teicoplanin were pumped through the column at 0.76 BV/h. After adsorption was finished, adsorbent was rinsed with 27 BV of demineralized water (1.14 BV/h), 25 BV of 15 vol.% methanol (1.14 BV/h), 6.4 BV of demineralized water (1.9 BV/h), 0.56 BV of demineralized water pH=2 (1.9 BV/h) and eluted with 50 vol.% methanol/pH=2 (0.38 BV/h). Two fractions were collected, 1700 mL with 14.8 g/L teicoplanin and 100 area % chromatographic purity and 5560 mL with 11 g/L teicoplanin and 98.29 area % of chromatographic purity (Table 2).

50 g of activated carbon Norit CA1™ were added to 7260 mL of the combined fractions. The suspension was vigorously stirred for two hours and then activated carbon was filtered off. The remaining filtrate was neutralized with sodium hydroxide solution from pH 2.8 to pH 6.6 and concentrated to 1000 mL by means of vacuum evaporation (T<40°C). To precipitate teicoplanin, acetone was continuously added to the concentrate while stirring until the ratio of acetone:concentrate was approximately 11:1 . The resulting suspension was filtered over a ceramic filter (porosity B4) to recover a precipitate, which was washed twice with 100 mL of acetone and then dried in a vacuum dryer at 40 °C for 20 hours to yield 60.3 g of brownish teicoplanin powder with chromatographic purity of 98.07 area %.

**Table 2**

| | | **Fraction 1** | **Fraction 2** | **Precipitate** |
|---|---|---|---|---|
| **TA3-1** | % area | 17.04 | 1.27 | 7.82 |
| **TA2 main component** | % area | 55.63 | 89.08 | 75.89 |
| **TA2 other components** | % area | 27.33 | 7.93 | 14.36 |
| **TA2 total** | % area | 82.96 | 97.02 | 90.25 |
| **TA2 + TA3-1** | % area | **100** | **98.29** | **98.07** |
| **Impurities > Rt TA2-5** | % area | **0** | **1.71** | **1.93** |

The table shows that a skilled person may collect fractions taking into consideration a balance between economy of the process and technical simplicity on one side and regulatory demands including safety for patients on the other side. He can collect limited crops with extreme purity of close to 100 % with no detectable related impurities. He can further reduce portions of insufficient fractions by using a longer column or he can use insufficient fractions for an additional cycle of column treating in the next batch.

### EXAMPLE 12

### Teicoplanin permeability through ultrafiltration membranes in different methanol/water mixtures

Removal of coloured impurities from a solution of crude teicoplanin crystals, prepared according to Example 11 (5 g/L) in water/methanol mixture was tested in a stirred ultrafiltration cell Amicon 8050 (trademark of Millipore) on various membranes with a cut-off of 5, 10, 20, 50 and 100 kDa, selected from commercial membranes of trademark Alfa Laval. In different experiments methanol/water mixtures of 0, 20, 40, 60, 80 v/v % ratio were tested on permeability. 50 mL of solution were added to the stirred cell, filtration was initiated by pressurization of the cell with nitrogen, and a magnetic stirrer was set to high speed to reduce effects of concentration polarization. The permeate was collected in a measuring cylinder. Filtration was stopped when a volumetric concentration factor (VCF) of approximately 5 was reached. Samples of the permeate and of the concentrate were taken and analyzed by HPLC. The results of the experiments are summarized in Table 3.

**Table 3**

| **Membrane type** | **MWCO** | **C MeOH** | **C₀** | **dP** | **T** | **VCF** | **R** | **Permeate colour** |
|---|---|---|---|---|---|---|---|---|
| - | kDa | vol.% | g/L | bar | °C | - | - | - |
| **UF X5 pHt** | 5 | 20 | 5 3 | 3 0 | 20 | 5 6 | 092 | colourless |
| | | 50 | 5 5 | 3 0 | 21 | 5 3 | 0 50 | colourless |
| | | 80 | 5 4 | 3 0 | 20 | 5 1 | 0 31 | colourless |
| **GR 81 PP** | 10 | 20 | 5 7 | 3 0 | 21 | 5 0 | 0 94 | colourless |
| | | 50 | 5 5 | 3 0 | 20 | 5 0 | 075 | colourless |
| | | 80 | 5 8 | 2 0 | 29 | 5 3 | 063 | slightly yellowish |
| **GR 61 PP** | 20 | 80 | 6 0 | 2 0 | 21 | 5 0 | 0 16 | slightly yellowish |
| **GR 51 PP** | 50 | 0 | 5 4 | 2 0 | 20 | 5 4 | 0 89 | colourless |
| | | 80 | 5 7 | 3 0 | 20 | 5 6 | 002 | light yellow |
| **GR 40 PP** | 100 | 0 | 5 5 | 3 0 | 20 | 6 1 | 0 88 | colourless |

### EXAMPLE 13

### Purification of teicoplanin by ultrafiltration

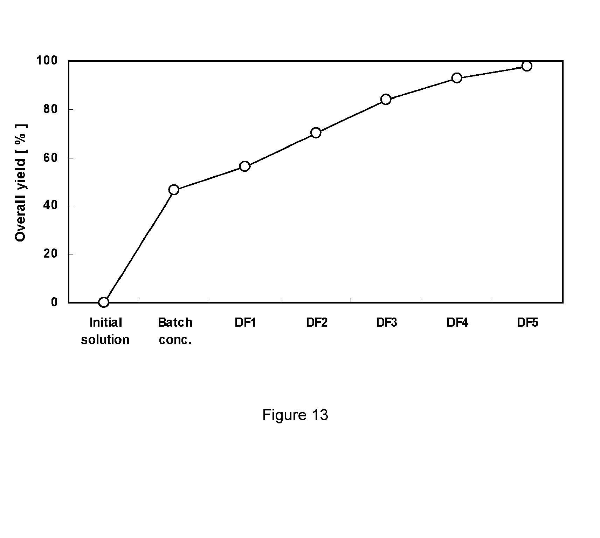

50 mL of solution of teicoplanin (water-methanol mixture, 2.8 g teicoplanin/L, pH 7.3), prepared by collection of chromatography fractions after desorption of teicoplanin from Amberlite XAD1180™ as described in the first paragraph of example 11 were added into a stirred UF cell Amicon 8050™ (Millipore, MA) with 5 kDa molecular weight cut-off ultrafiltration membrane (trademark of Alfa Laval®) and filtration was initiated by pressurization of the cell with nitrogen. A magnetic stirrer was set to high speed (200 rpm) to reduce effects of concentration polarization. The permeate was collected in a measuring cylinder. 5-fold batch concentration was followed by five diafiltrations, each with 40 mL of 80 vol. % MeOH. Samples of the permeate and of the concentrate were taken and analyzed by HPLC. The overall yield of the batch concentration and diafiltration together was >95% (Figure 13).

### EXAMPLE 14

### Preparation of teicoplanin powder

50L of teicoplanin broth permeate from fermentation culture of *Actinoplanes teichomyceticus,* containing 2.1 g of teicoplanin were pumped through a column of 4.9 cm of diameter, loaded with Amberlite XAD-1180™ of 90 cm of bed height. After adsorption the resin was rinsed with 30BV demineralized water, 25 BV 15 vol.% methanol, 10 BV demineralized water and 1 BV of diluted HCl with pH of 2. Teicoplanin was eluted from the adsorbent with 50 vol.% methanol with pH adjusted to 2 with 10 % (w/w) HCl. Four fractions were collected (total 7.6 BV) and purity of teicoplanin was determined by HPLC analysis. The first fraction (0.5 BV) was discarded, the second teicoplanin-rich fraction (4.1 BV, 11.8 g/L teicoplanin) with overall coloured oxidized impurities content 2.36 area % was purified further, the the third fraction (1.5 BV, 5.2 g/L teicoplanin) with overall coloured oxidized impurities content 6.72 area % was kept for optional repurification. The fourth fraction with overall coloured oxidized impurities content >8.5 area % 1.5 BV) was discarded.

The main fraction was transferred into a beaker with a mechanical stirrer and 0.6 g of activated carbon Norit CA1™ per g of teicoplanin were added. The mixture was vigorously stirred for 2 hours and the charcoal was filtered off. The resulting filtrate (6.2 L) was neutralized to pH 6.5 with aqueous 1 % (w/w) sodium hydroxide. The neutralized filtrate was further purified by a crossflow ultrafiltration (SEPA CF II, GE Osmonics, MN™) utilizing 5 kDa molecular weight cut-off flat sheet ultrafiltration membrane UF X5 pHt (Alfa Laval) with 155 cm2 filtration area. The feed solution was first concentrated batchwise 6-fold, followed by six diafiltrations with 1.5 L of 80 % (v/v) MeOH each. The transmembrane pressure drop was kept at 2.5-5 bars and the temperature of the concentrate at 23-25°C. The permeate (14.2 L, 4.3 g/L teicoplanin) was concentrated by evaporation under vaccum in a rotavapor to obtain the concentrate with approximately 80 g/L teicoplanin. The concentrate (0.76 L) was further transferred to a 10 L container and 7 L of acetone were added dropwise while the resulting mixture was vigorously stirred. The obtained suspension was stirred for further 2 hours and then the precipitate was filtered off and dried in a vacuum dryer at 40 °C to give 55.8 g of slightly yellowish powder with purity of 98.13 area %.

## Claims

1. A process for preparing purified teicoplanin, the process comprising the steps of
a) adsorbing teicoplanin to a hydrophobic adsorption resin;
b) desorbing teicoplanin from said resin using a medium containing water and a water-miscible organic solvent;
c) treating a solution comprising teicoplanin with charcoal followed by charcoal removal;
d) subjecting a solution comprising teicoplanin to a process of ultrafiltration on membranes with a cut-off of 3-100 kDa, wherein the solution contains more than 20 % (v/v) of a water-miscible organic solvent and not more than 90 % (v/v) of a water-miscible organic solvent;
e) optionally concentrating and/or desalinating a teicoplanin-containing solution after ultrafiltration defined in step d) by ultrafiltration on membranes with a cut-off of 1000 Da;
f) obtaining purified teicoplanin.

2. The process according to claim 1, wherein the steps a) to f) are performed in the order as indicated in claim 1.

3. The process according to claims 1 or 2, further comprising in advance a step of adding an organic solvent to a culture of *Actinoplanes teichomyceticus,* and removal of mycelium and other insoluble material by filtration and/or centrifugation.

4. The process according to any one of claims 1 or 3, wherein the hydrophobic adsorption resin is a polystyrene or polyacrylate crosslinked non-ionic macroreticular resin having a pore size of 20 to 500 A.

5. The process according to any one of claims 1 to 4, wherein the hydrophobic adsorption resin is selected from the group consisting of Hypersol-Macronet®, Dowex® Optipore, Amberlite® XAD16, Amberlite® XAD 1600, Amberlite® XAD 1180, Diaion® HP20, Diaion® Sepabeads SP207 and Dianion® HP2MG.

6. The process according to any one of claims 1 to 5, wherein the step of desorbing teicoplanin from the hydrophobic adsorption resin is performed using an aqueous medium comprising at least 40 % (v/v) of a water-miscible organic solvent.

7. The process according to any one of claims 1 to 6, wherein the water-miscible organic solvent is selected from C₁-C₄ alcohols, C₃-C₄ ketones or C₂-C₄ nitriles.

8. The process according to any one of claims 1 to 7, wherein the step of desorbing teicoplanin from the hydrophobic adsorption resin is performed using an aqueous medium having a pH value below 4.

9. The process according to claim 8, wherein the pH value of the aqueous medium is adjusted by addition of an aqueous solution of a mineral acid, an organic acid or an acidic buffer.

10. The process according to any one of claims 1 to 9, wherein the medium of the solution undergoing the charcoal treatment originates from step b) and comprises at least 40 % (v/v) of an organic solvent, selected from the group of C₁-C₄ alcohols, C₃-C₄ ketones or C₂-C₄ nitriles.

11. The process according to any one of claims 1 to 10, wherein the solution obtained in step b) comprises 60 to 80 % (v/v) of methanol or is corrected by addition of fresh solvent in order to have a methanol concentration of 60 to 80 % (v/v).

12. The process according to any one of claims 1 to 11, wherein the ultrafiltration of step d) is performed on a solution obtained in step c), which comprises more than 20 % (v/v) and up to 90 % (v/v) of a C₁-C₄ alcohol, or on a solution obtained in step c), which has been corrected by addition of fresh solvent in order to comprise more than 20 % (v/v) and up to 90 % (v/v) of a C₁-C₄ alcohol.

13. The process according to any one of claims 1 to 12, wherein the ultrafiltration of step d) is performed using a membrane with a cut-off of 3 - 100 kDa, preferably of 5 - 100 kDa, more preferably of 5 - 10 kDa, and/or wherein the ultrafiltration of step e) is performed using membranes with a cut-off of 1000 Da.

14. The process according to any one of claims 1 to 13, wherein obtaining purified teicoplanin in step f) is performed by teicoplanin isolation involving removal of the solvent using evaporation, vacuum evaporation, liophilisation, spray drying or precipitation by adding antisolvent.

15. A process for manufacturing a pharmaceutical composition comprising the process according to any one of claims 1 to 14, and wherein purified teicoplanin is admixed with at least one pharmaceutical excipient or carrier.
